# EUROPEAN PATENT SPECIFICATION

(11) **EP 2 947 181 B1**
(45) Date of publication and mention of the grant of the patent: **22.02.2017**
(21) Application number: 14169722.7
(22) Date of filing: 23.05.2014
(51) Int. Cl.: C25D 1/08, A61M 15/00, B05B 17/00, A61M 11/00

(54) **A METHOD FOR PRODUCING AN APERTURE PLATE**
VERFAHREN ZUR HERSTELLUNG EINER LOCHPLATTE
PROCÉDÉ DE PRODUCTION D'UNE PLAQUE PERFORÉE

(43) Date of publication of application: 25.11.2015
(73) Proprietor: STAMFORD DEVICES LIMITED, Dangan Galway (IE)
(72) Inventor: Hogan, Brendan, County Galway (IE); Xu, Hong, Redwood City, CA 94065 (US)
(74) Representative: Weldon, Michael James

(56) References cited:
- JP-A- 2002 019 125
- US-A1- 2006 203 036
- US-B2- 7 066 398
- L. Vecellio: "The mesh nebuliser : a recent technical innovation for aerosol delivery", Breathe, Vol. 2, N° 3, March 2006 (2006-03), XP055151396, Retrieved from the Internet: URL:http://www.ers-education.org/lrmedia/2 006/pdf/40403.pdf [retrieved on 2014-11-06]

## Description

### Introduction

The invention relates to manufacture of aperture plates (or "vibrating membranes") for aerosol (or "nebulizer") devices.

An aperture plate is used for aerosol delivery of liquid formulations delivering a controlled liquid droplet size suitable for pulmonary drug delivery. The ideal nebulizer is one which assures a consistent and accurate particle size in combination with an output rate that can be varied to deliver the drug to the targeted area as efficiently as possible. Delivery of the aerosol to the deep lung such as the bronchi and bronchiole regions requires a small and repeatable particle size typically in the range of 2 - 4 µm. In general, outputs greater than 1 ml/min are required.

US6235177, US7066398 and US8398001 describe an approach to manufacturing an aperture plate in which a wafer is built onto a mandrel by a process of electro-deposition where the dissolved metal cations in the plating bath (typically Palladium and Nickel) are reduced using electrical current from the liquid form to the solid form on the wafer. Material is reduced only to the conducting surface on the mandrel and not to photo resist islands which are non-conducting. The aperture hole size and profile are defined by the three dimensional growth of plated materials, thus, electroforming. After the conclusion of the plating process, the mandrel/wafer assembly is removed from the bath and the wafer peeled from the mandrel for subsequent processing into an aperture plate.

However, a problem with this approach is that the wafer thickness is intertwined with the placement density of the non-conducting resist islands, i.e. thicker the wafer, further the distance between the non-conduction resist islands, and the smaller the aperture density. As a result, it is not possible to increase the aperture density to about 90,000 holes per square inch (approx.. 650mm²). Reduced wafer thickness is used to alleviate this issue. If a wafer has a thickness in the range of 50-54µm non-standard drives are required. Also, a reduction in the aperture plate thickness alters the harmonics and natural frequency of the core assembly. To drive this optimally to generate acceptable flow rate output, it is necessary to alter the drive frequency. This is disadvantageous as it requires the development, manufacture and supply of a matching drive controller which has consequential cost and lead time implications.

Photo-defined technology as described in WO2012/092163A and WO2013/186031A allows a large number of holes to be produced in a very small area because it applies standard wafer processing technology to define the aperture plate hole size and profile through photolithography, thus, "photo-defined".

This technology may involve plating multiple layers to achieve desired aperture plate geometry and profile, for example, a first layer normally called the outlet or droplet size defining layer, and subsequently imparting a second layer on top of this which is normally called the inlet or reservoir layer. It can be challenging to achieve the correct levels of interfacial adhesion between both layers. And the process is more complex than that for electroforming.

US2006/0203036 (Sexton et al) describes an annular nozzle structure for high density printheads.

JP2002019125 (Hitachi) describes manufacture by electroforming of a nozzle body.

The invention is directed towards providing an improved method to address the above problems.

### Summary of the Invention

According to the invention, there is provided a method of manufacturing an aperture plate wafer, as set out in claim 1.

In one embodiment, the columns have a height in the range of 55µm to 65µm. In one embodiment, the column width dimension is in the range of 20 µm to 40 µm, preferably 25 µm to 35 µm.

In one embodiment, the combined wafer plate achieved by the column height and the height of over-plating is in the range of 50 µm to 70 µm. Preferably, the aperture size is in the range of 2µm to 6µm.

In one embodiment, the wafer is formed into a dome shape which is concave on the side of the apertures.

In one embodiment, at least some columns have a generally convex top surface.

In one embodiment, the columns are configured so that when the masking material is removed they form passageways aligned with the apertures and being shaped for entrainment of droplets from the apertures.

In one embodiment, at least some of the columns have a configuration widening towards the substrate so that after removal of the masking material they form passageways which widen in a direction away from the apertures. Preferably, the passageways are gradually tapered with a consistent slope. In one embodiment, the plated metal includes Ni. In one embodiment, the plated metal includes Pd. In one embodiment, both Ni and Pd are present in the plated metal.

In one embodiment, both Ni and Pd are present in the plated metal; and wherein the proportion of Pd is in the range of 85% w/w and 93% w/w, and preferably about 89%, substantially the balance being Ni.

In one embodiment, the method comprises the further steps of further processing the wafer to provide an aperture plate ready to fit into an aerosol-forming device. In one embodiment, the wafer is punched and formed into a non-planar shaped aperture plate. In one embodiment, the wafer is annealed before punching.

In another aspect, the invention provides an aperture plate comprising a body of metal configured with aerosol-forming apertures in a top surface and passageways aligned with and beneath the apertures, wherein the passageways have a length of 40µm to 70µm, the aperture plate has a thickness in the range of 50µm to 70µm, the passageways have a width in the range of 20 µm to 40 µm, and the aperture size is in the range of 2µm to 6µm.

In one embodiment, the metal body forms convex shapes around the apertures to provide a funnel-shaped entrance to the apertures. In one embodiment, the passageways widen towards the lower side of the plate.

In one embodiment, the passageways are gradually tapered with a uniform sloped taper.

In one embodiment, the passageways have a length of 55µm to 65µm.

In one embodiment, the passageways have a width in the range of 25 µm to 35 µm.

An aerosol-forming device comprising an aperture plate as defined above in any embodiment.

### Detailed Description of the Invention

The invention will be more clearly understood from the following description of some embodiments thereof, given by way of example only with reference to the accompanying drawings in which:-
Figs. 1 to 4 are a series of cross-sectional views showing stages of manufacturing an aperture plate;
Fig. 5 is a cross-sectional view of a stage in an alternative embodiment;
Fig. 6 is a cross-sectional diagram illustrating an alternative approach in which there are tapered openings below the aerosol-forming apertures; and
Fig. 7 shows an aperture plate of the type n Fig. 6 after it has been formed into a dome shape.

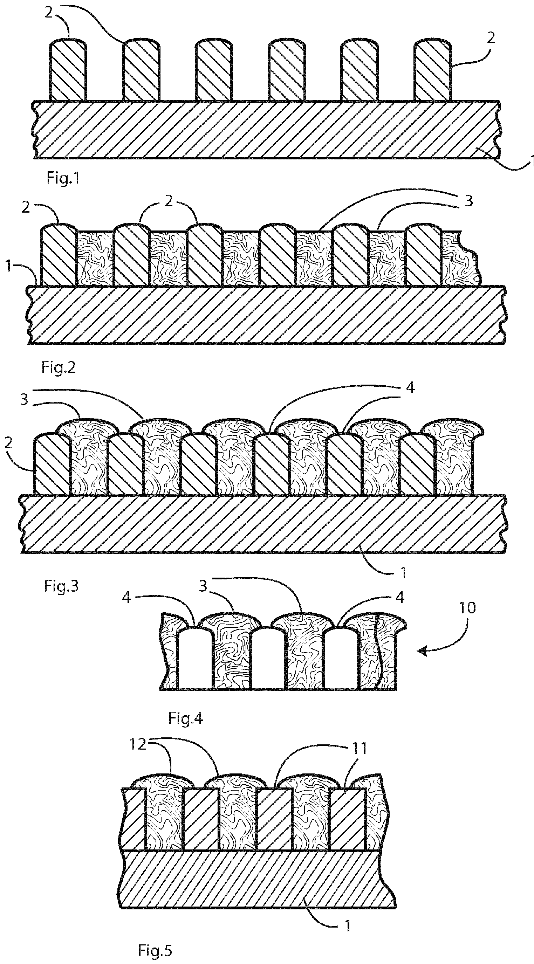

Referring to Figs. 1 to 4, the following are the main steps to manufacture an aperture plate in one embodiment.

An aperture plate is manufactured by plating metal around a mask of resist columns 2 having a height of about 45µm, a diameter of about 30 µm and a separation of about 30 µm,. The plating is continued so that the metal 3 overlies the top surfaces of the columns until the desired apertures 4 are achieved. This provides the benefits of both photo-defined technology (by reducing the aspect ratio near the aperture region during electroforming) and aperture density by enabling more closely patterned resist islands, with need for only one masking/plating cycle to achieve the desired plate thickness.

In more detail, non-conductive photo-resist 2 is laid on to a mandrel 1 substrate. This is developed to leave the upstanding columns 2 where holes are required. The tops of these columns are approximately convex. The mandrel is placed in an electroforming tank. As the plating continues, the space between the columns 2 of developed photo resist are in-filled with the plating material. This is typically a PdNi alloy matrix, or it could alternatively be Nickel or a Nickel Cobalt alloy matrix.

The plating is initially to the extent shown in Fig. 2 and is continued so that over plating occurs as shown in Fig. 3.This plating is stopped just in time to create 2 - 6 µm holes 4 as shown also in Fig. 3.

The diametrical size accuracy of these holes can be improved by slowing down the plating deposition activity as the holes are being formed. This prevents 'overshoot' resulting in smaller or occluded holes with the possibility of a thicker than desired wafer construction. The 45 micron column height is so chosen such that when the plating is stopped (Fig. 3) the holes are typically 2 - 6 µm and preferably 2 - 5 µm, which is required to produce droplets in the inhalable range for nebulisation and concurrently the wafer thickness is in the range of 60 - 62 µm.

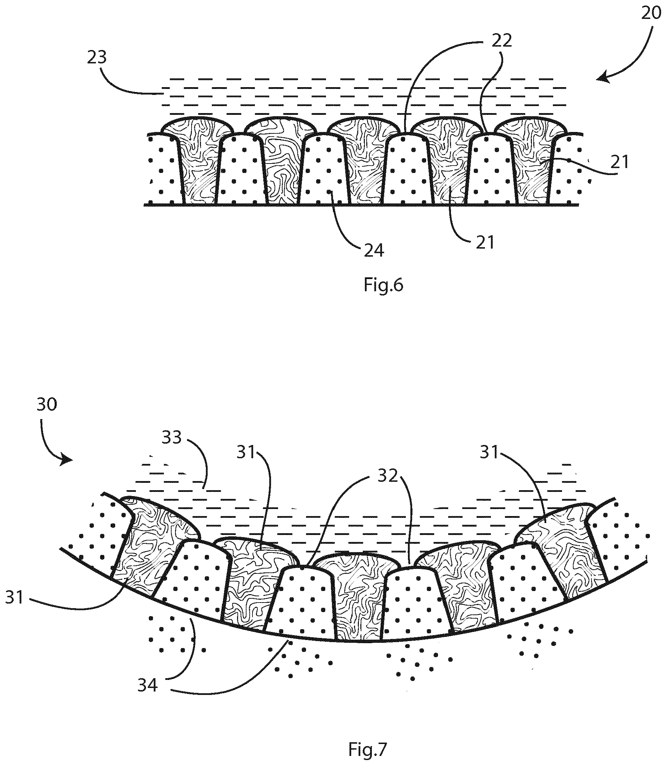

The convex shape of the entry surfaces to the apertures in addition to the concave shape of the overall domed shaped aperture plate (Fig. 7) provides effective funnelling of the liquid towards the aerosol-forming apertures 4, thereby minimising the residual volume of the drug in the nebuliser. When the photo-resist 2 is removed using an appropriate dissolving solvent, the full wafer cross-section is evident as depicted in Fig. 4. The cross-sectional profile of the hole formations provided by resist removal under the apertures encourages entrainment (Fig. 7) of the aerosol towards the outlet of the nebuliser thereby reducing coalescence with the resultant undesirable effect of larger droplets being formed.

In an alternative embodiment (Fig. 5), photo-resist columns 11 have flat top surfaces over which the metal (12) is plated.

As evident from Fig. 6, in a plate 20 the remaining metal may form outlet hole or passageway 24 sides that are tapered towards the aerosol outlet direction. This drawing shows the wafer metal 21 forming aerosol-forming apertures 22. The liquid 23 is aerosolized through the apertures 22 to exit as droplets through the entrainment openings or passageways 24 aligned with and below the apertures 22.

Fig. 7 shows an aperture plate 30 with metal 31 forming apertures 32 and droplet entrainment openings 34, after being formed into a dome shape. As noted above, this dome shape together with the convex shape of the metal between the apertures 32 helps to effectively funnel the liquid 33 towards the apertures 32 in order to form droplets, which exit via the entrainment openings 34.

These much larger holes 34 in comparison (to the aperture diameter) can entrain the aerosol, almost into a laminar flow pattern. This reduces turbulence and consequential coalescence which can lead to an undesirable increase in droplet size. These openings may be tapered (Figs. 6 and 7) or not (Figs 1-4).

The resultant wafer 10 has a greater number of holes, greater than 6835 per square centimetre (44,100 per square inch (Mesh 210)), than the prior art and yet maintains the same aperture plate thickness (approximately 61µm) as the standard commercially available products. This ensures that the existing drive controllers (128 kHz) already *in situ* in many hospitals can be used for the aperture plate, alleviating the cost and considerable time required to be expended to develop a bespoke drive controller to ensure that the correct frequency is available to achieve optimum aerosol output. It is also more conducive for meeting and exceeding the fatigue life requirements. As there is single-layer plating it incorporates a fine equiaxed microstructure.

It will be appreciated that the method provides the benefits of both photo-defined technology, partially decoupling the dependence of wafer thickness to resist island patterning distance and increased aperture density, with the process simplicity of electroforming, because it needs only one masking/plating cycle to achieve the desired plate thickness.

Those skilled in the electro-deposition field will appreciate how the plating conditions may be chosen to suit the circumstances, and the entire contents of the following documents are herein incorporated by reference: US4628165, US6235117, US2007023547, US2001013554,

WO2009/042187, and Lu S. Y., Li J. F., Zhou Y. H., "Grain refinement in the solidification of undercooled Ni-Pd alloys", Journal of Crystal Growth 309 (2007) 103-111, Sept 14 2007. Generally, most electroplating solutions involving Palladium and Nickel would work or Nickel only or indeed Phosphorous & Nickel (14:86) or Platinum. It is possible that a non-Palladium wafer could be plated at the surface (1 - 3 microns thick) in PdNi to impart more corrosion resistance. This would also reduce the hole sizes if smaller openings were desired.

The resist geometry, such as, height, width and shape, is configured in such a way to increase the number of holes while maintaining the desired wafer thickness. Further increase of hole density is also possible. For example, the invention in one embodiment achieves an aperture plate of about 4 times the density (moving from 210 to 420 holes per linear inch or from 44,100 to 176,400 holes per square inch), while still maintaining the typical 60 to 62 µm thickness range.

Adjusting the dimensions in Fig. 1, by reducing the column 2 diameter (30 µm) and dimensions between the columns 2 to say 15 µm has the potential to increase the number of holes to 700 - 850 per linear inch

The invention avoids need for two-layer photo defined technology to increase the number of holes while maintaining the same wafer thickness. It also solves the problem of using standard plating defined technology with a greater number of holes which will result in a lower thickness wafer thus requiring significant changes to the core construction or more typically the drive controller to find the optimum drive frequency.

The invention finds particular application where faster nebulisation treatment times are required. This is usually required for hand-held devices when aerosol is administrated through the mouth or nasal passages in fully mobile patients. These are typically patients who administer nebulised drugs in a non-hospital setting.

This is in contrast to intubated hospital patients who are typically on mechanical ventilation where treatment times are less important as long as the patient gets the full prescribed dose.

Techniques for vibrating the aperture plates are described generally in U.S. Pat. Nos. 5,164,740; 5,586,550; and 5,758,637, which are incorporated herein by reference. The aperture plates are constructed to permit the production of relatively small liquid droplets at a relatively feast rate.

For example, the aperture plates of the invention may be employed to produce liquid droplets having a size in the range from about 2 microns to about 10 microns, and more typically between about 2 microns to about 5 microns. In some cases, the aperture plates may be employed to produce a spray that is useful in pulmonary drug delivery procedures. As such, the sprays produced by the aperture plates may have a respirable fraction that is greater than about 70%, preferably more than about 80%, and most preferably more than about 90% as described in U.S. Pat. No. 5,758,637.

In some embodiments, such fine liquid droplets may be produced at a rate in the range from about 2 microliters per second to about 25 microliters per second per 1000 apertures. In this way, aperture plates may be constructed to have multiple apertures that are sufficient to produce aerosolized volumes that are in the range from about 2 microliters to about 25 microliters, within a time that is less than about one second. Such a rate of production is particularly useful for pulmonary drug delivery applications where a desired dosage is aerosolized at a rate sufficient to permit the aerosolised medicament to be directly inhaled. In this way, a capture chamber is not needed to capture the liquid droplets until the specified dosage has been produced. In this manner, the aperture plates may be included within aerosolisers, nebulizers, or inhalers that do not utilise elaborate capture chambers.

The aperture plate may be employed to deliver a wide variety of drugs to the respiratory system. For example, the aperture plate may be utilized to deliver drugs having potent therapeutic agents, such as hormones, peptides, and other drugs requiring precise dosing including drugs for local treatment of the respiratory system. Examples of liquid drugs that may be aerosolized include drugs in solution form, e.g., aqueous solutions, ethanol solutions, aqueous/ethanol mixture solutions, and the like, in colloidal suspension form, and the like. The invention may also find use in aerosolizing a variety of other types of liquids, such as insulin.

It will be appreciated that the invention allows the production of a wafer from which nebuliser aperture plates are punched in one single plating step and facilitates the creation of a larger number of holes than that known today (typically up to 400%). Also, it facilitates the use of aperture plates which are 60-62 µm thick. Also, it allows an increase in the number of holes per unit of area while still being able to control the plating thickness to a predetermined dimension. The above in combination allows the creation of a higher output nebuliser while still maintaining the standard drive controller and core construction all of which is accomplished in a very economical manner.

## Claims

1. A method of manufacturing an aperture plate wafer, the method comprising providing a substrate (1) of conductive material, applying a mask (2) over the substrate in a pattern of columns extending from the substrate and having top surfaces, electroplating (3) spaces around the columns (2), removing the mask to provide a wafer of the electroplated material with aerosol-forming holes (4) where the mask columns were,
**characterized in that**, the electroplating step partially over-plates (3) the column top surfaces while leaving aerosol-forming apertures (4) of a desired size, and with a density of greater than 6835/cm², and the columns have a height in the range of 40µm to 70µm.

2. The method as claimed in claim 1, wherein the columns have a height in the range of 55µm to 65µm.

3. The method as claimed in claims 1 or 2, wherein the column width dimension is in the range of 20µm to 40µm, and preferably 25µm to 35µm.

4. The method as claimed in any preceding claim, wherein the combined wafer plate (10, 20, 30) achieved by the column height and the height of over-plating is in the range of 50 µm to 70 µm.

5. The method as claimed in any preceding claim, wherein the aperture (4) size is in the range of 2µm to 6 µm.

6. The method as claimed in any preceding claim, wherein the wafer is formed into a dome shape (30) which is concave on the side of the apertures.

7. The method as claimed in any preceding claim, wherein at least some columns (2) have a generally convex top surface.

8. The method as claimed in any preceding claim, wherein the columns (2, 11) are configured so that when the masking material is removed they form passageways (24, 34) aligned with the apertures and being shaped for entrainment of droplets form the apertures.

9. The method as claimed in any preceding claim, wherein at least some of the columns have a configuration widening towards the substrate so that after removal of the masking material they form passageways (24, 34) which widen in a direction away from the apertures with a consistent slope.

10. An aperture plate (10, 20, 30) comprising a body of metal configured with aerosol-forming apertures (4, 22, 32) in a top surface and passageways (24, 34) aligned with and beneath the apertures,
wherein the passageways have a length of 40µm to 70µm, the aperture plate has a thickness in the range of 50µm to 70µm, the passageways have a width in the range of 20 µm to 40 µm, and the aperture size is in the range of 2µm to 6µm

11. The aperture plate as claimed in claim 10, wherein the metal forms convex shapes (3, 21, 31) around the apertures to provide a funnel-shaped entrance to the apertures.

12. The aperture plate as claimed in claims 10 or 11, wherein the passageways (24, 34) widen towards the lower side of the plate.

13. The aperture plate as claimed in any of claims 10 to 12, wherein the passageways have a length of 55µm to 65µm.

14. The aperture plate as claimed in any of claims 10 to 13, wherein the passageways have a width in the range of 25µm to 35µm.

15. An aerosol-forming device comprising an aperture plate of any of claims 10 to 14.

## Patentansprüche

1. Verfahren zum Herstellen eines Lochplattenwafers, wobei das Verfahren Folgendes umfasst: Bereitstellen eines Substrats (1) aus leitfähigem Material, Aufbringen einer Abdeckung (2) über dem Substrat in einem Muster von Säulen, die sich von dem Substrat erstrecken und obere Oberflächen aufweisen, Galvanisieren (3) von Zwischenräumen um die Säulen (2) herum, Entfernen der Abdeckung, um einen Wafer des galvanisierten Materials mit Aerosol bildenden Löchern (4), wo sich die Abdeckungssäulen befanden, zu bilden,
**dadurch gekennzeichnet, dass** der Galvanisierungsschritt die oberen Oberflächen der Säulen teilweise übergalvanisiert (3) und dabei Aerosol bildende Löcher (4) einer gewünschten Größe und mit einer Dichte von mehr als 6835/cm² hinterlässt, und die Säulen eine Höhe im Bereich von 40 µm bis 70 µm aufweisen.

2. Verfahren nach Anspruch 1, wobei die Säulen eine Höhe im Bereich von 55 µm bis 65 µm aufweisen.

3. Verfahren nach Anspruch 1 oder 2, wobei das Säulenbreitenmaß im Bereich von 20 µm bis 40 µm, und vorzugsweise 25 µm bis 35 µm liegt.

4. Verfahren nach einem der vorangehenden Ansprüche, wobei die durch die Säulenhöhe und die Höhe des Übergalvanisierens erreichte kombinierte Waferplatte (10, 20, 30) im Bereich von 50 µm bis 70 µm liegt.

5. Verfahren nach einem der vorangehenden Ansprüche, wobei die Größe der Löcher (4) im Bereich von 2 µm bis 6 µm liegt.

6. Verfahren nach einem der vorangehenden Ansprüche, wobei der Wafer zu einer Kuppelform (30) geformt wird, die auf der Seite der Löcher konkav ist.

7. Verfahren nach einem der vorangehenden Ansprüche, wobei mindestens einige Säulen (2) eine allgemein konvexe obere Oberfläche aufweisen.

8. Verfahren nach einem der vorangehenden Ansprüche, wobei die Säulen (2, 11) derart ausgebildet sind, dass, wenn das Abdeckmaterial entfernt wird, sie Durchgänge (24, 34) bilden, die auf die Löcher ausgerichtet sind und zum Mitreißen von Tröpfchen von den Löchern gestaltet sind.

9. Verfahren nach einem der vorangehenden Ansprüche, wobei mindestens einige der Säulen eine Ausbildung aufweisen, die zum Substrat hin breiter wird, sodass sie nach dem Entfernen des Abdeckmaterials Durchgänge (24, 34) bilden, die in einer Richtung von den Löchern weg mit einer gleichbleibenden Steigung breiter werden.

10. Lochplatte (10, 20, 30), umfassend einen Körper aus Metall, der mit Aerosol bildenden Löchern (4, 22, 32) in einer oberen Oberfläche und Durchgängen (24, 34), die auf die Löcher ausgerichtet sind und sich unter ihnen befinden, ausgebildet ist,
wobei die Durchgänge eine Länge von 40 µm bis 70 µm aufweisen, die Lochplatte eine Dicke im Bereich von 50 µm bis 70 µm aufweist, die Durchgänge eine Breite im Bereich von 20 µm bis 40 µm aufweisen und die Lochgröße im Bereich von 2 µm bis 6 µm liegt.

11. Lochplatte nach Anspruch 10, wobei das Metall konvexe Formen (3, 21, 31) um die Löcher herum bildet, um einen trichterförmigen Eingang zu den Löchern bereitzustellen.

12. Lochplatte nach Anspruch 10 oder 11, wobei die Durchgänge (24, 34) zur unteren Seite der Platte hin breiter werden.

13. Lochplatte nach einem der Ansprüche 10 bis 12, wobei die Durchgänge eine Länge von 55 µm bis 65 µm aufweisen.

14. Lochplatte nach einem der Ansprüche 10 bis 13, wobei die Durchgänge eine Breite im Bereich von 25 µm bis 35 µm aufweisen.

15. Aerosol bildende Vorrichtung, die eine Lochplatte nach einem der Ansprüche 10 bis 14 umfasst.

## Revendications

1. Procédé de fabrication d'une tranche à plaque perforée d'ouvertures, le procédé comportant les étapes consistant à mettre en oeuvre un substrat (1) de matériau conducteur, appliquer un masque (2) sur le substrat selon un motif de colonnes s'étendant depuis le substrat et ayant des surfaces supérieures, électrogalvaniser (3) les espaces autour des colonnes (2), retirer le masque pour la mise en oeuvre d'une tranche du matériau électrogalvanisé avec des trous de formation d'aérosol (4) là où les colonnes du masque étaient,
**caractérisé en ce que**, l'étape d'électrogalvanisation effectue un surdépôt partiel (3) au niveau des surfaces supérieures des colonnes tout en laissant des ouvertures de formation d'aérosol (4) d'une taille souhaitée, et avec une densité de plus de 6835/cm², et les colonnes ont une hauteur dans la plage allant de 40 µm à 70 µm.

2. Procédé selon la revendication 1, dans lequel les colonnes ont une hauteur dans la plage allant de 55 µm à 65 µm.

3. Procédé selon la revendication 1 ou la revendication 2, dans lequel la dimension de largeur de colonne est dans la plage allant de 20 µm à 40 µm, et de préférence de 25 µm à 35 µm.

4. Procédé selon l'une quelconque des revendications précédentes, dans lequel la plaque de tranches combinées (10, 20, 30) réalisée par la hauteur de la colonne et la hauteur du surdépôt est dans la plage allant de 50 µm à 70 µm.

5. Procédé selon l'une quelconque des revendications précédentes, dans lequel la taille des ouvertures (4) est dans la plage allant de 2 µm à 6 µm.

6. Procédé selon l'une quelconque des revendications précédentes, dans lequel la tranche est réalisée en une forme de dôme (30) qui est concave du côté des ouvertures.

7. Procédé selon l'une quelconque des revendications précédentes, dans lequel au moins certaines colonnes (2) ont une surface supérieure généralement convexe.

8. Procédé selon l'une quelconque des revendications précédentes, dans lequel les colonnes (2, 11) sont configurées de telle sorte que, quand la matière de masquage est retirée, elles forment des voies de passage (24, 34) qui sont alignées sur les ouvertures et formées à des fins d'entraînement de gouttelettes en provenance des ouvertures.

9. Procédé selon l'une quelconque des revendications précédentes, dans lequel au moins certaines des colonnes ont une configuration allant en s'élargissant vers le substrat de telle sorte que, après le retrait de la matière de masquage, elles forment des voies de passage (24, 34) qui vont en s'élargissant dans une direction allant à l'opposé des ouvertures avec une inclinaison uniforme.

10. Plaque perforée d'ouvertures (10, 20, 30) comportant un corps de métal configuré avec des ouvertures de formation d'aérosol (4, 22, 32) dans une surface supérieure et des voies de passage (24, 34) alignées sur les ouvertures et sous celles-ci,
dans laquelle les voies de passages ont une longueur de 40 µm à 70 µm, la plaque perforée d'ouvertures a une épaisseur dans la plage allant de 50 µm à 70 µm, les voies de passage ont une largeur dans la plage allant de 20 µm à 40 µm, et la taille des ouvertures est dans la plage allant de 2 µm à 6 µm.

11. Plaque perforée d'ouvertures selon la revendication 10, dans laquelle le métal réalise des formes convexes (3, 21, 31) autour des ouvertures pour la mise en oeuvre d'une entrée en forme d'entonnoir donnant sur les ouvertures.

12. Plaque perforée d'ouvertures selon la revendication 10 ou la revendication 11, dans laquelle les voies de passage (24, 34) vont en s'élargissant vers le côté inférieur de la plaque.

13. Plaque perforée d'ouvertures selon l'une quelconque des revendications 10 à 12, dans laquelle les voies de passage ont une longueur de 55 µm à 65 µm.

14. Plaque perforée d'ouvertures selon l'une quelconque des revendications 10 à 13, dans laquelle les voies de passage ont une largeur dans la plage de 25 µm à 35 µm.

15. Dispositif de formation d'aérosol comportant une plaque perforée d'ouvertures selon l'une quelconque des revendications 10 à 14.
